# EUROPEAN PATENT APPLICATION

(11) **EP 3 815 678 A1**
(43) Date of publication of application: **05.05.2021**
(21) Application number: 19824794.2
(22) Date of filing: 27.06.2019
(51) Int. Cl.: A61K 9/107, A61K 9/66, A61K 9/70, A61K 38/18, A61K 47/42, A61K 47/44

(54) **GEL-IN-OIL EMULSION AND TRANSDERMALLY ABSORBED AGENT**

(30) Priority: 29.06.2018 JP 2018124249
(71) Applicant: Nippon Shokubai Co., Ltd., Osaka-shi, Osaka 541-0043 (JP); Kyushu University, National University Corporation, Nishi-ku Fukuoka-shi Fukuoka 819-0395 (JP)
(72) Inventor: IJIMA Hiroyuki, Fukuoka-shi, Fukuoka 819-0395 (JP); SHIRAKIGAWA Nana, Fukuoka-shi, Fukuoka 819-0395 (JP); YAMAMOTO Emiko, Fukuoka-shi, Fukuoka 819-0395 (JP)
(74) Representative: Hoffmann Eitle
(86) International application number: PCT/JP2019/025749
(87) International publication number: WO 2020/004599

(57) **Abstract**

A gel-in-oil emulsion comprising a gelled water-soluble polymer, an oil, and a surfactant, and a transdermal absorption preparation comprising the gel-in-oil emulsion.

## Description

### Technical Field

The present invention relates to a gel-in-oil emulsion and a transdermal absorption preparation.

### Background Art

A transdermal absorption preparation is intended to allow an active ingredient such as a drug to be absorbed from the skin and to be introduced into the body. Herein, in order to allow an active ingredient to be transdermally absorbed, it is necessary for the active ingredient to pass through the hydrophobic stratum corneum that is a surface layer of the skin and to be delivered to the inside of the hydrophilic skin. For this reason, a transdermal absorption preparation is required to have hydrophilic-hydrophobic control.

In this regard, for example, in Patent Literature 1, it has been reported that a predetermined water-in-oil microemulsion is useful as a transdermal absorption preparation (a drug delivery system)

### Citation List

### Patent Literature

Patent Literature 1: Japanese Unexamined Patent Publication No. H11-29464

### Summary of Invention

### Technical Problem

Incidentally, it is preferred that a transdermal absorption preparation has a sustained-release property for the purpose of stably maintaining the concentration of the active ingredient in the blood over a long period of time. However, for a conventional water-in-oil (W/O) microemulsion disclosed in Patent Literature 1 or the like, there has been the following problem: release of an active ingredient from the transdermal absorption preparation is rapid, and thus the effect does not last long.

Therefore, it is an object of the present invention to provide a transdermal absorption preparation with an excellent sustained-release property, and a gel-in-oil emulsion to be used for the transdermal absorption preparation.

### Solution to Problem

In view of the circumstances described above, the present inventors, as a result of intensive studies, have completed the inventions indicated in the following [1] to [6].
[1] A gel-in-oil emulsion, comprising a gelled water-soluble polymer, an oil, and a surfactant.
[2] The gel-in-oil emulsion according to [1], containing a water-soluble active ingredient in the gelled water-soluble polymer.
[3] The gel-in-oil emulsion according to [2], wherein the water-soluble active ingredient is a growth factor.
[4] The gel-in-oil emulsion according to any of [1] to [3], wherein the gelled water-soluble polymer is a gelled polymer derived from a biological organism.
[5] The gel-in-oil emulsion according to [4], wherein the polymer derived from a biological organism is a collagen or a gelatin.
[6] A transdermal absorption preparation, comprising the gel-in-oil emulsion according to any of [1] to [5].

### Advantageous Effects of Invention

According to the present invention, a transdermal absorption preparation with an excellent sustained-release property, and a gel-in-oil emulsion to be used for the transdermal absorption preparation can be provided.

### Brief Description of Drawings

Fig. 1 is a schematic cross-sectional view of a device used for skin permeability evaluation in Examples.
Fig. 2 shows photographs indicating the results of phase difference observation and fluorescence observation after the skin permeability test in Example 1 and Comparative Example 1.
Fig. 3 shows photographs of HE staining observation in Example 6 and Comparative Example 5.
Fig. 4 is a graph showing the results of measurement of absorbance in Example 6 and Comparative Example 5.

### Description of Embodiments

Hereinafter, one embodiment of the present invention will be described in detail, but the present invention is not limited thereto.

A gel-in-oil emulsion (hereinafter, also referred to as "G/O emulsion") of the present embodiment comprises a gelled water-soluble polymer, an oil, and a surfactant. The G/O emulsion of the present embodiment may also comprise a water-soluble active ingredient and the like in the gelled water-soluble polymer, as needed. In this regard, the G/O emulsion is an emulsion in which gel particles derived from a gelled water-soluble polymer are dispersed in oil.

The average particle diameter of the gel particles in the emulsion is preferably 1 µm or less, more preferably 800 nm or less, furthermore preferably 600 nm or less, still more preferably 400 nm or less, and particularly preferably 200 nm or less. By setting the average particle diameter of the gel particles in the above range, the permeation into normal vascular tissue is suppressed, but the permeability to inflammatory vascular tissue is maintained, and thus the active ingredient can be delivered more efficiently, and this is more preferred also from the viewpoint of safety. The lower limit value of the average particle diameter of the gel particles is not particularly limited, and can be set to, for example, 30 nm or more or 50 nm or more. In this regard, the average particle diameter of the gel particles is a particle diameter measured by a dynamic light scattering method.

Hereinafter, each component in the G/O emulsion of the present embodiment will be described in detail.

### (Gelled water-soluble polymer)

A water-soluble polymer is a polymer at least part of which is dissolved in ion exchanged water at normal temperature, and is more specifically, for example, a polymer of which 1 g or more of a sample is dissolved in 100 mL of ion exchanged water. Specific examples of the water-soluble polymer include polymers derived from biological organisms such as water-soluble collagen, gelatin, fibronectin, laminin, chitin, chitosan, albumin, casein, fibroin, fibrin, vitronectin, hyaluronic acid, hyaluronic acid, hyaluronic acid ester, dextran, pullulan, agar, algic acid, starch, inulin, fucoidan, chitosan, or cellulose; and synthetic polymers such as polyvinyl alcohol, a polyacrylic acid-based polymer, polyethylene glycol, polyethylene oxide, polyvinyl pyrrolidone, polyacrylamide, polyoxazoline, polyglycidol, and copolymers thereof. Among them, polymers derived from biological organisms are preferred, and collagen or gelatin is more preferred. As the water-soluble polymer, these polymers may be used singly or in combinations of two or more.

In this regard, the water-soluble polymer can turn into a gel, for example, by keeping an aqueous solution of the water-soluble polymer at a low temperature or a high temperature. A water-soluble polymer derived from a biological organism such as gelatin has a random coil-shaped molecular structure in a heated solution, but when the solution is cooled, part of the gelatin molecule has a helical structure, a network is formed, and thus the gelatin turns into a gel. A water-soluble polymer such as water-soluble collagen undergoes self-association in a physiological environment (at a pH near neutral and 37°C), and turns into a gel. The gelling temperature varies depending on the type of the water-soluble polymer, and it is also one of the preferred embodiments to allow a water-soluble polymer gelation of which is dissolved at a temperature in the vicinity of body temperature or a water-soluble polymer that cannot turn into a gel due to temperature to turn into a gel by crosslinking between molecules of the water-soluble polymer through chemical crosslinking.

Examples of the method of chemical crosslinking include a method of using a crosslinking agent, and a method of irradiation with a ultraviolet light or an electron beam.

The method of using a crosslinking agent can be applied to preferably a water-soluble polymer having an amino group and/or a carboxyl group, more preferably a water-soluble polymer having an amino group and a carboxyl group, and furthermore preferably a water-soluble polymer which is derived from a biological organism and has an amino acid-derived structure.

The crosslinking agent is not particularly limited as long as it can prevent the toxicity to the living body. Specific examples of the crosslinking agents include aldehydes such as glutaraldehyde or formaldehyde; carbodiimides such as dicyclohexyl carbodiimide (DCC), diisopropyl carbodiimide (DIC), 1-ethyl-3-(3-dimethylaminopropyl)carbodiimide hydrochloride (EDC), or 1-cyclohexyl-3-(2-morpholinoethyl)carbodiimide metho-p-toluenesulfonate; and multiply-charged ions of chromium, aluminum, iron, or the like. Among them, glutaraldehyde or 1-ethyl-3-(3-dimethylaminopropyl)carbodiimide hydrochloride is preferred.

The chemical crosslinking may be performed between water-soluble polymers, or may be performed by introducing another compound having an amino group, a carboxyl group or the like (for example, heparin), which can be reacted with a water-soluble polymer, and using the compound.

The content of the water-soluble polymer is, for example, preferably 0.001 to 50% by mass, and more preferably 0.1 to 20% by mass, based on the total amount of the G/O emulsion.

In this regard, the gelled water-soluble polymer contains water derived from an aqueous solution in addition to the water-soluble polymer. The content of the water is preferably 30 to 99% by mass, and more preferably 50 to 98% by mass, based on the total amount of the water-soluble polymer and water.

### (Oil)

Examples of the oil include vegetable oils such as soybean oil, cottonseed oil, rapeseed oil, sesame oil, corn oil, peanut oil, safflower oil, sunflower oil, olive oil, rapeseed oil, perilla oil, fennel oil, cacao oil, cinnamon oil, mentha oil, or bergamot oil; animal oils such as beef tallow, lard, or fish oil; fatty acid triglycerides such as ethylhexyl triglyceride, or medium-chain triglyceride being C8, C10 fatty acid mixed triglyceride; non-volatile hydrocarbons such as liquid paraffin, squalene, squalane, or pristane; and ester compounds of a lower alcohol and a higher fatty acid, such as isopropyl myristate, octyldodecyl myristate, cetyl myristate, ethyl oleate, ethyl linoleate, isopropyl linoleate, isopropyl palmitate, or butyl stearate. Among them, ester compounds of a lower alcohol and a higher fatty acid are preferred, and isopropyl myristate or isopropyl palmitate is more preferred. As the oil, these oils may be used singly or in combination of two or more.

The content of the oil is, for example, preferably 50 to 99.9% by mass, and more preferably 75 to 99.7% by mass, based on the total amount of the G/O emulsion.

### (Surfactant)

As the surfactant, there is an anionic, a cationic, an amphoteric, or a nonionic surfactant. Each surfactant has a hydrophilic group and a hydrophobic group in the molecule, and is classified as hydrophilic or hydrophobic depending on the balance (HLB value) of the hydrophilic group and the hydrophobic group. Specifically, a surfactant having an HLB value of more than 10 is classified as hydrophilic, and a surfactant having an HLB value of 10 or less is classified as hydrophobic. These surfactants can be used without any particular limitation, and as the surfactant, a hydrophobic surfactant is preferred, and a hydrophobic nonionic surfactant is more preferred. These surfactants may be used singly or in combination of two or more.

Examples of the hydrophobic nonionic surfactants include sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, sorbitan sesquioleate, sorbitan monoisostearate, sorbitan monolaurate, penta-2-ethylhexyl acid diglycerol sorbitan, and tetra-2-ethylhexyl acid diglycerol sorbitan; glycerine fatty acid esters such as glycerol monostearate, glycerol monooleate, and glyceryl monostearate malic acid; and polyglycerol fatty acid esters such as diglyceryl tetraisostearate, diglyceryl diisostearate, diglyceryl monoisostearate, polyglyceryl monostearate, polyglyceryl monoisostearate, polyglyceryl diisostearate, polyglyceryl monolaurate, polyglyceryl monooleate, and polyglyceryl monomyristate.

The content of the surfactant is, for example, preferably 0.05 to 20% by mass, more preferably 0.05 to 15% by mass, and furthermore preferably 0.1 to 15% by mass, based on the total amount of the gel particles in the G/O emulsion (including the water and the water-soluble active ingredient in the gel).

### (Water-soluble active ingredient)

A water-soluble active ingredient is a component of which at least part is dissolved in ion exchanged water at normal temperature, and more specifically, may be any that is dissolved in such an ion exchanged water to the extent that the effect attributable to the water-soluble active ingredient can be exerted in the body. Any substance, as long as it has such a property, can be appropriately selected depending on the application without any limitation on the fields of cosmetics, pharmaceuticals, quasi drugs, foods, and the like. Further, the water-soluble active ingredient may be a synthetic substance, or a natural substance. These may be used singly or in combination of two or more. Specific examples of the water-soluble active ingredient include growth factors or the like such as hepatic parenchymal cell growth factor (HGF), epidermal growth factor (EGF), fibroblast growth factor (FGF), vascular endothelial growth factor (VEGF), nerve growth factor (NGF), tumor growth factor (TGF), bone morphogenetic protein (BMP), keratinocyte growth factor (KGF), insulin-like growth factor (IGF), hepatocyte growth factor (HGF), T-cell growth factor, lymphokines, or cytokines; water-soluble hormones such as insulin; axon guidance molecules such as netrin; water-soluble drugs such as various kinds of low-molecular-weight therapeutic agents, a protein preparation, an enzyme drug, or high-molecular-weight therapeutic agents such as DNA; moisturizing components, anti-inflammatory agents, convergent components, vitamins, peptides, amino acids, antimicrobial components, horny softening components, cell activation components, anti-aging components, blood circulation promoting action components, whitening components, and hair growth agents.

In this regard, in a case where, for example, a growth factor is contained in a gelled water-soluble polymer, it is preferred to allow heparin to coexist in the gelled water-soluble polymer. When heparin is allowed to coexist in the gelled water-soluble polymer, the growth factor in the water-soluble polymer forms a heparin-growth factor complex to immobilize the growth factor. In this way, the growth factor in the water-soluble polymer is more stabilized, and the function can be more effectively exhibited. Further, it is preferred that the water-soluble polymer is a water-soluble polymer having an amino group. By activating a carboxyl group in the heparin with N-hydroxysuccinimide (NHS), N-hydroxysulfosuccinimide sodium (Sulfo-NHS), 1-hydroxybenzotriazole (HOBT) or the like and condensing the carboxyl group with the amino group in the water-soluble polymer, in the presence of a condensing agent, for example, carbodiimide such as dicyclohexyl carbodiimide (DCC), diisopropyl carbodiimide (DIC), or 1-ethyl-3-(3-dimethylaminopropyl)carbodiimide hydrochloride (EDC), the heparin can be immobilized in the water-soluble polymer. In this way, the growth factor can be more stabilized in the water-soluble polymer.

It is preferred that the content of water-soluble active ingredient is a concentration at which the water-soluble active ingredient exerts an effective effect as a transdermal absorption preparation. The content of the water-soluble active ingredient is, for example, preferably 50% by mass or less, and more preferably 10% by mass or less, based on the total amount of the G/O emulsion. In this regard, the lower limit of the content of the water-soluble active ingredient is not particularly limited, and can be set to, for example, 1 ppm by mass or more.

### (Method for producing G/O emulsion)

The method for producing the G/O emulsion is not particularly limited, and the G/O emulsion can be prepared, for example, by the method described below.

First, a second liquid containing an aqueous solution of a water-soluble polymer is added to a first liquid containing an oil and a surfactant to obtain a mixture. This mixture is dispersed in an oil, and further the obtained dispersion is, for example, cooled and allowed to turn into a gel by a sol-gel change or the like, and a G/O emulsion in which emulsion particles containing a gelled water-soluble polymer are dispersed in an oil is obtained. Further, it is also one of the preferred embodiments to perform the gelation by chemical crosslinking when a water-soluble polymer is dispersed in an oil. Examples of the method for dispersion include an intermittent shaking method, a method by a propeller-type stirrer, a turbine-type stirrer, or a mixer such as a disperser, a colloid mill method, a homogenizer method, and an ultrasonic irradiation method. In addition, as needed, the emulsion particles and the oil are separated by applying decantation, centrifugation, filtration, or the like, and a G/O emulsion in which emulsion particles are redispersed in an oil prepared separately can also be formed. The more preferred separation method is separation by centrifugation. By performing such a processing, impurities dissolved in the oil contained in the raw material can be removed. In this regard, the oil contained in the first liquid and the oil used for the redispersion may be the same or may be different from each other.

By adding the water-soluble active ingredient to the second liquid, a water-soluble active ingredient can also be contained in the gelled water-soluble polymer.

Further, by mixing the above-described G/O emulsion with a surfactant and water which are used for secondary emulsification, and dispersing and emulsifying the obtained mixture with an external aqueous phase component, a G/O/W multi-phase emulsion in which an active ingredient is retained in an internal aqueous phase can also be prepared. By using the G/O/W multi-phase emulsion, a G/O emulsion including a water-soluble active ingredient can be dispersed in water, the stickiness due to the oil can be reduced, and the usability can be improved.

### (Transdermal absorption preparation)

The transdermal absorption preparation of the present embodiment contains the above-described G/O emulsion. The transdermal absorption preparation may contain the G/O emulsion as the above-described G/O/W multi-phase emulsion. The transdermal absorption preparation contains a G/O emulsion, and therefore, is excellent in the sustained-release property. The dosage form of the transdermal absorption preparation may be a dosage form with which the state as the G/O emulsion can be maintained, and examples of the dosage form include various types of dosage forms for external use, such as a liquid (including a lotion and a spray), an ointment, a cream, a gel, an emulsifying liquid, and a stick agent. Further, it is also one of the preferred embodiments to use the G/O emulsion of the present embodiment as a patch preparation by adding a dispersion of the G/O emulsion into a pressure-sensitive adhesive or a thermoplastic elastomer to prepare a coating liquid, and applying the coating liquid to a support and/or a release liner.

In a case of using as the transdermal absorption preparation, the transdermal absorption preparation can be used with the addition of a transdermal absorption promoting agent, an antioxidant, a fragrance, a coloring agent, and the like, as needed. Further, in a case of using as the patch preparation, the patch preparation can be used with the further addition of a tackifying agent, an excipient, and the like. In the patch preparation, the content of the G/O emulsion based on the total amount of the G/O emulsion and the tackifying agent is preferably 10% by mass or more, more preferably 25% by mass or more, and furthermore preferably 40% by mass or more.

The patch preparation can be produced, for example, by a method including the following steps (1) to (3):
(1) a step of preparing a G/O emulsion;
(2) a step of increasing the concentration of the G/O emulsion by concentrating the G/O emulsion, removing the supernatant, and the like; and
(3) a step of adding an adhesiveness imparting agent and an additive agent to the G/O emulsion to prepare a coating liquid, and applying the coating liquid to a support or a release liner.

The amount of the transdermal absorption preparation of the present embodiment to be used differs depending on the type of disease, the severity of symptoms, and the size of the affected part, and cannot be specified unconditionally. The transdermal absorption preparation is usually used once or several times a day by applying an adequate amount to the affected part.

### Examples

Hereinafter, the present invention will be described in more detail by way of Examples, but the present invention should not be limited only by these Examples.

### (Preparation Example 1)

### [Preparation of EGFP (enhanced green fluorescent protein)]

EGFP-introduced E. coli (BL21) was cultured, isopropyl-P-galactopyranoside (manufactured by Sigma-Aldrich Co. LLC.) was added to the cultured BL21, and the obtained preparation was induced for 4 hours for expression. The E. coli was collected, the culture medium was replaced with a sonication buffer adjusted to pH 8.0 (a mixture of 50 mM, Tris aminomethane (manufactured by Sigma), 50 mM NaCl (manufactured by Wako Pure Chemical Industries, Ltd.), 1 mM EDTA (manufactured by Wako Pure Chemical Industries, Ltd.), and 1 mM Dithiotheitol (manufactured by Sigma)), sonication was performed, and the supernatant was used in the following experiments.

### (Example 1)

### [Preparation of EGFP-containing G/O emulsion]

A liquid obtained by mixing 700 µl of the EGFP prepared in Preparation Example 1 with 300 µl of 10% gelatin solution (Gelatin from porcine skin, manufactured by Sigma-Aldrich Co. LLC) was referred to as liquid A, and a liquid obtained by dissolving 125 mg of a hydrophobic surfactant, ER-290 (trade name, manufactured by MITSUBISHI-CHEMICAL FOODS CORPORATION, sucrose erucic acid ester) in 2.5 ml of isopropyl myristate (IPM manufactured by Wako Pure Chemical Industries, Ltd.) was referred to as liquid B. 1 ml of liquid A was mixed with 2.5 ml of liquid B, and the obtained mixture was subjected to treatment for 10 minutes by using an ultrasonic homogenizer. The resultant mixture was cooled at 4°C for 30 minutes to obtain an emulsion. The obtained emulsion was centrifuged at 5000 rpm for 1 minute, and then the supernatant was removed by suction. Into the resultant emulsion, 500 µl of IPM was added to redisperse the emulsion therein, and an EGFP-containing G/O emulsion was prepared.

### [Skin permeability evaluation using mouse skin]

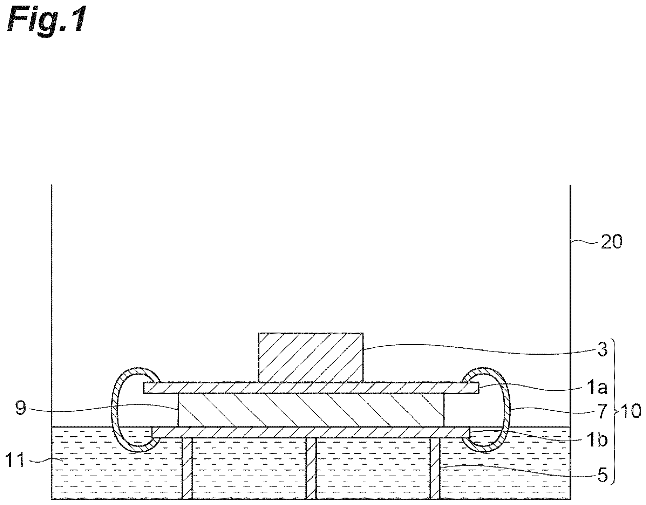

By using a device shown in Fig. 1, skin permeability was evaluated. The device shown in Fig. 1 is provided with a diffusion cell 10 having holding parts 1a and 1b for holding a mouse skin 9, a sample addition port 3 arranged on the holding part 1a side, a support part 5 arranged on the holding part 1b side, and a connection part 7 that connects the holding parts 1a and 1b. This diffusion cell 10 was installed in the bottom part of one well 20 of a 6-well plate such that the support part 5 was in contact with the bottom part, and phosphate-buffered saline 11 was added to the well 20 to the extent that the phosphate-buffered saline 11 was in contact with a lower surface of the mouse skin 9, and the resultant device was used for evaluation. Hereinafter, a specific operation method will be described.

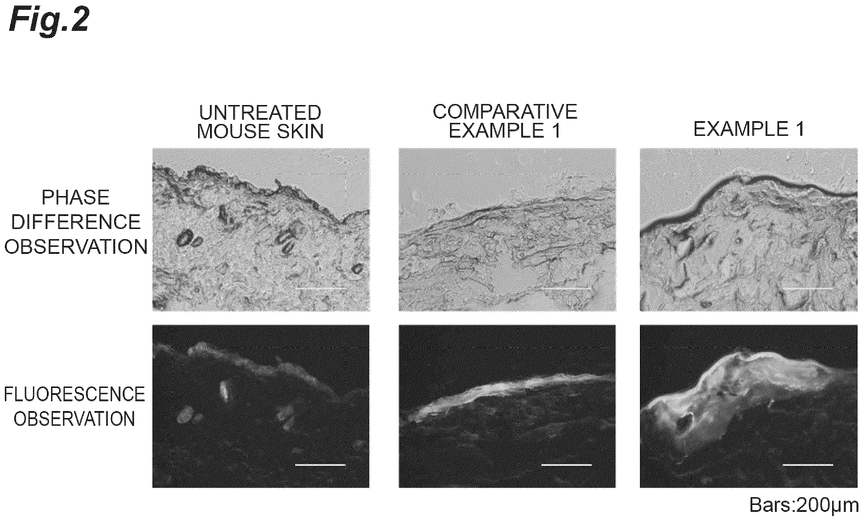

After the hair on the back of a mouse was lightly shaved with a razor, the remaining hair was cleanly removed by using a depilatory, and the skin was washed with ultrapure water. The whole layer of the skin of the mouse was excised, and cut into a piece having an around 2 cm square with scissors. The skin was sandwiched between the holding parts of the diffusion cell shown in Fig. 1. The diffusion cell was placed in one well of a 6-well plate (manufactured by Thermo Scientific), and PBS (phosphate-buffered saline) was added into the one well to the extent that the PBS was in contact with a lower surface of the skin. 200 µl of the EGFP-containing G/O emulsion described above was added from the sample addition port. A permeability test was performed by placing the plate in an incubator and leaving the plate to stand at 37°C for one day. The skin was recovered, and the surface of the skin was wiped lightly with PROWIPE. The skin was cut into small pieces, the piece was embedded in OCT Compound (manufactured by Sakura Finetek Japan Co., Ltd.), then the embedded piece was frozen at -80°C, and a tissue slice having a thickness of 20 µm was prepared with a microtome. The tissue slice was adsorbed on a slide glass, and the phase difference observation and the fluorescence observation were performed. The results are shown in Fig. 2.

### (Comparative Example 1)

A permeability test was performed in a similar manner as in Example 1 except that the EGFP prepared in Preparation Example 1 was added from the sample addition port, and the phase difference observation and fluorescence observation of the skin after the test were performed. The results are shown in Fig. 2.

As is obvious from Fig. 2, it is confirmed that the EGFP permeated into the deeper part of the skin in a case where the EGFP-containing G/O emulsion was used (Example 1) as compared with the case where EGFP alone was used (Comparative Example 1).

### (Synthesis Example 1)

### [Preparation of L929 cell-embedded collagen gel]

Ultrapure water was added to a collagen made of pig skin (manufactured by NH Foods Ltd.), and the obtained mixture was stirred overnight at 4°C to prepare a 1% collagen solution. AL929 cell suspension (obtained by collecting cultured L929 cells and suspending the collected L929 cells in a culture medium) (6×10⁵ cells) was centrifuged at 1000 rpm for 3 minutes, and the supernatant was removed by suction. A 20% fetal bovine serum (Fetal Bovine Serum, manufactured by FBS bio sera) was added to a Dulbecco's Modified Eagle Medium (DMEM) (manufactured by Thermo Fisher Scientific Inc.) prepared at a concentration of twice to prepare a 2-fold concentrated DMEM culture medium. L929 cells were suspended in a solution in which the 1% collagen solution and the 2-fold concentrated DMEM culture medium were mixed at a ratio of 1:1. This suspension was added to a 12-well plate at 1 ml/well, and was left to stand in an incubator for 1 hour to allow the suspension to turn into a gel.

### (Example 2)

### [Preparation of bFGF-containing G/O emulsion]

Into a 50 mM MES buffer (manufactured by Wako Pure Chemical Industries, Ltd.), heparin (manufactured by Wako Pure Chemical Industries, Ltd.), 1-(3-dimethylaminopropyl)-3-ethylcarbodiimide hydrochloride (EDC, manufactured by PEPTIDE INSTITUTE, INC.), and N-hydroxysuccinimide (NHS, manufactured by Wako Pure Chemical Industries, Ltd.) were added so as to have a mass ratio of 10:10:6 to prepare a heparin/EDC/NHS solution having a heparin concentration of 10 mg/ml, and then the heparin/EDC/NHS solution was left to stand for 30 minutes at room temperature. 990 µl of 5% gelatin solution was mixed with 10 µl of the heparin/EDC/NHS solution. Further, 25 µl of 10 µg/ml bFGF (basic fibroblast growth factor, manufactured by R&D Systems, Inc.) was added to the mixture to obtain a liquid referred to as liquid C. A liquid in which 50 mg of a hydrophobic surfactant, ER-290 (trade name, manufactured by MITSUBISHI-CHEMICAL FOODS CORPORATION, sucrose erucic acid ester) was dissolved in 2.0 ml of IPM was referred to as liquid D. 1 ml of liquid C was mixed with 2 ml of liquid D, and then the obtained mixture was subjected to treatment for 3 minutes by using an ultrasonic homogenizer. Next, the resultant mixture was cooled at 4°C for 30 minutes to obtain an emulsion. The obtained emulsion was centrifuged at 5000 rpm for 1 minute, and then the supernatant was removed by suction. Into the resultant emulsion, 500 µl of IPM was added to redisperse the emulsion therein to prepare a bFGF-containing G/O emulsion.

### [Titer evaluation using cultured dermis model]

A ring made of polycarbonate (outer diameter: 1.4 cm, inner diameter: 1.2 cm, and height: 0.3 cm) was placed on a L929 cell-embedded collagen gel (cultured dermis model) prepared in Synthesis Example 1. 200 µl of the bFGF-containing G/O emulsion described above was added to the inside the ring. 200 µl of DMEM culture medium was added to the outside of the ring, and the gel was placed in an incubator. The culture medium was changed every day, and the amounts of DNA in the L929 cell-embedded collagen gel after 1 day, 3 days, and 5 days were quantified. The quantification of DNA was performed as follows.

A liquid in which 1 ml of 0.25% collagenase solution (manufactured by Wako Pure Chemical Industries, Ltd.) was added to a L929 cell-embedded collagen gel was referred to as liquid E. A liquid in which a gel was dissolved by shaking the gel at 37°C for around 1 hour was referred to as liquid F. Each sample was centrifuged at 1000 rpm for 3 minutes, and the supernatant was removed by suction. Triton X-100 (manufactured by Sigma) was added to the above-described PBS to prepare 0.1% Triton X-100-containing PBS, and the sample was suspended in 1 ml of the 0.1% Triton X-100-containing PBS. The obtained suspension was frozen at -80°C, and then thawed at room temperature. This operation was repeated 3 times. 180 µl of Hoechst (Hoechst (registered trademark), manufactured by Thermo Fisher Scientific K.K.) was mixed with 20 µl of sample, and the obtained mixture was added in a 96-well black plate. By using Fluorescent DNA Quantitation Kit Cat. #170-2480 (BIO-RAD), the quantification of DNA was performed with fluorescent brightness measurement under the conditions of an excitation filter of 355 nm and an absorption filter of 460 nm. The fluorescent brightness was measured 3 times, and the average was taken to obtain the amount of DNA. The quantification of DNA was performed before the start of the test (0 day), and 1 day, 3 days, 5 days, and 7 days after the start of the test. The results are shown in Table 1.

### (Comparative Example 2)

A bFGF-containing W/O emulsion was prepared in a similar manner as in Example 2 except that water was used in place of the 5% gelatin solution. The quantification of the amount of DNA was performed by using the cultured dermis model in a similar manner as in Example 2 except that the obtained bFGF-containing W/O emulsion was used. The results are shown in Table 1.

### (Comparative Example 3)

The quantification of the amount of DNA was performed by using the cultured dermis model in a similar manner as in Example 2 except that bFGF was used as it was. The results are shown in Table 1.

### (Comparative Example 4)

bFGF at a concentration of 10 µg/ml was diluted 1000 times with DMEM to prepare a culture medium containing bFGF at a concentration of 10 ng/ml. The quantification of the amount of DNA was performed by using the cultured dermis model in a similar manner as in Example 2 except that 200 µl of the obtained bFGF-containing culture medium was used. The results are shown in Table 1.

As is obvious from Table 1, when the bFGF-containing W/O emulsion was used (Comparative Example 2), the amount of DNA after 3 days was large, but the increase in the amount of DNA was reduced after 5 days or later. This is considered that in the early stage of culture, a large amount of bFGF was released at one time from the W/O emulsion, the concentration in the culture medium was increased, and the proliferation was promoted, but the stability of the bFGF was extremely poor, the bFGF was depleted with the lapse of time, and as a result, the increase in the amount of cells was led to reduction after 5 days or later. When a large amount of active ingredient is released at one time in a living body, the active ingredient is rapidly diffused by the water and blood in the body, and the effective concentration at a place where the effect is desired to be exerted is substantially decreased, leading to a situation in which the effect does not last. On the other hand, in a case where the bFGF-containing G/O emulsion was used (Example 2), the amount of DNA after 3 days was smaller as compared with those in Comparative Examples 2, 3, and 4, but after 7 days, the amount of DNA was larger than that in any of Comparative Examples 2, 3 and 4. As a result, it can be understood that the bFGF-containing G/O emulsion is excellent in the sustained-release property of the bFGF, and the bFGF is stabilized. Accordingly, the following is expected: the active ingredient is stabilized and sustainably released also in the body; the active ingredient concentration becomes constant; and the effect is expected to last.

**[Table 1]**

| | Amount of DNA (ng) | | | | |
|---|---|---|---|---|---|
| | 0 Day | After 1 day | After 3 days | After 5 days | After 7 days |
| Example 2 | 43 | 90 | 125 | 201 | 279 |
| Comparative Example 2 | 43 | 138 | 151 | 168 | 205 |
| Comparative Example 3 | 43 | 74 | 145 | 187 | 192 |
| Comparative Example 4 | 43 | 65 | 150 | 210 | 159 |

### (Example 3)

150 mg of hydrophobic surfactant ER-290 was added to 2 ml of cyclohexane, the obtained mixture was further mixed with 1 ml of 5% gelatin sol, and the resultant mixture was subjected to treatment for 10 minutes by using an ultrasonic homogenizer. After the treatment, the mixture was cooled at 4°C for 3 days to obtain an emulsion. The obtained emulsion was centrifuged at 5000 rpm for 1 minute, and then the supernatant was removed by suction. IPM was added to redisperse the emulsion therein to make the concentration of the obtained particles 1 mg/ml, and thus a G/O emulsion was prepared. For the obtained G/O emulsion, the average particle diameter of the gel particles was measured by a dynamic light scattering method (Zetasizer Nano-ZS, manufactured by Marvern Panalytical Ltd.). The G/O emulsion was dispersed by using IPM (refractive index: 1.434), the dispersion was introduced into a glass cell, and by measuring the dispersion 5 times at a temperature of 20°C, the average of the peak values was taken to obtain the average particle diameter. The results are shown in Table 2.

### (Example 4)

A G/O emulsion was prepared in a similar manner as in Example 3 except that L-195 (manufactured by MITSUBISHI-CHEMICAL FOODS CORPORATION) was used as the surfactant. For the obtained G/O emulsion, the results obtained by measuring the average particle diameter of gel particles are shown in Table 2.

### (Example 5)

A G/O emulsion was prepared in a similar manner as in Example 3 except that O-170 (manufactured by MITSUBISHI-CHEMICAL FOODS CORPORATION) was used as the surfactant. For the obtained G/O emulsion, the results obtained by measuring the average particle diameter of gel particles are shown in Table 2.

**[Table 2]**

| | Average particle diameter |
|---|---|
| Example 3 | 161 nm |
| Example 4 | 353 nm |
| Example 5 | 233 nm |

As is obvious from Table 2, in any G/O emulsion, the average particle diameter of gel particles was 600 nm or less.

### (Example 6)

### [Preparation of bFGF-containing G/O emulsion -2]

Into 50 mM MES buffer (manufactured by Wako Pure Chemical Industries, Ltd.), heparin (manufactured by Wako Pure Chemical Industries, Ltd.), 1-(3-dimethylaminopropyl)-3-ethylcarbodiimide hydrochloride (EDC, manufactured by PEPTIDE INSTITUTE, INC.), and N-hydroxysuccinimide (NHS, manufactured by Wako Pure Chemical Industries, Ltd.) were added so as to have a mass ratio of 10:10:6 to prepare a heparin/EDC/NHS solution having a heparin concentration of 10 mg/ml, and then the heparin/EDC/NHS solution was left to stand for 30 minutes at room temperature. 495 µl of 5% gelatin solution was mixed with 5 µl of heparin/EDC/NHS solution, and further 25 µl of 10 µg/ml bFGF solution (basic fibroblast growth factor, manufactured by R&D Systems, Inc.) was added thereto to obtain a liquid referred to as liquid C-2. A liquid in which 25 mg of a hydrophobic surfactant, ER-290 (trade name, manufactured by MITSUBISHI-CHEMICAL FOODS CORPORATION, sucrose erucic acid ester) was dissolved in 1.0 ml of IPM was referred to as liquid D-2. 500 µl of liquid C-2 was mixed with 1 ml of liquid D-2, and then the obtained mixture was subjected to treatment for 3 minutes by using an ultrasonic homogenizer. Next, the resultant mixture was cooled at 4°C for 30 minutes to obtain an emulsion. The obtained emulsion was centrifuged at 5000 rpm for 2 minutes, and then the supernatant was removed by suction to obtain a bFGF-containing G/O emulsion having a heparin concentration of 50 µg/ml and a bFGF concentration of 250 ng/ml.

### [Evaluation of angiogenesis inducing effect using mouse]

After anesthetizing a hairless mouse with isoflurane, the back of the hairless mouse was sterilized with 70% ethanol, and then, the sterilized part was washed with a sterilized saline solution. Next, around 1 ml of the G/O emulsion prepared in [preparation of bFGF-containing G/O emulsion -2] was applied onto the sterilized back of the hairless mouse, and a sterilized gauze having a size of 1.5 × 3.0 cm and moistened with 250 µl of IPM was put on the back and was stuck with a surgical bandage tape. After curing for 2 days, the gauze was removed, and the back of the hairless mouse was washed with a sterilized saline solution. The operations of application and the curing for 2 days were repeated three more times, then the skin in a test part of the mouse was excised and used as a sample, and the HE staining observation and measurement of hemoglobin index shown below were performed.

### [HE (hematoxylin-eosin) staining observation]

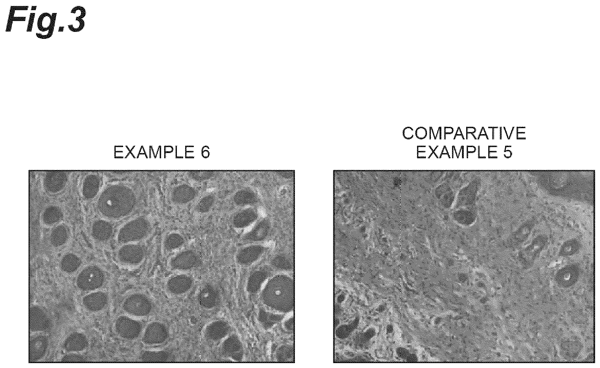

The sample of the excised skin was stained with a HE liquid in accordance with a conventional method, and the stained sample was observed with an optical microscope at 10-fold magnification. The results are shown in Fig. 3.

### [Measurement of hemoglobin index]

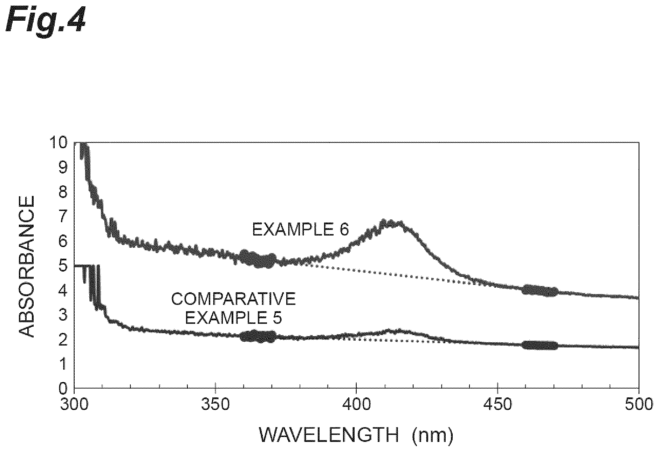

The skin on the back of the test mouse was excised, the excised skin was washed with a large amount of saline, and the blood adhered to the skin surface was washed off. Next, the washed skin was shredded with scissors, the shredded skin was transferred to a sample tube of 1.5 ml, 1 ml of ultrapure water was added into the sample tube, and the resultant sample tube was left to stand at 4°C for 1 day. After the filtration with a filter of 40 µm, the absorbance of the filtrate was measured. The absorbance of hemoglobin in blood vessels contained in the skin tissue has a peak at a wavelength in the vicinity of 415 nm, and therefore, the baseline of the absorbance of from 360 nm to 470 nm was approximated by a straight line, and the increment from the baseline of the absorbance at a wavelength of 415 nm was taken as the absorbance of hemoglobin in the sample (see Fig. 4). The thus obtained absorbance of hemoglobin in the skin sample was converted to a numerical value per weight of the skin sample, and the converted numerical value was taken as the hemoglobin index. The results are shown in Table 3. It is considered that the larger hemoglobin index indicates higher induction of angiogenesis.

### (Comparative Example 5)

The same operation as in Example 6 was performed except that the G/O emulsion was not applied, and the HE staining observation and the measurement of hemoglobin index were performed. The results are shown in Fig. 3, Fig. 4, and Table 3.

**[Table 3]**

| | Example 6 | Comparative Example 5 |
|---|---|---|
| Hemoglobin index | 8.76 | 2.59 |

As is obvious from Table 3, the hemoglobin index in Example 6 was higher than that in Comparative Example 5, and it is indicated that angiogenesis was induced by applying the bFGF-containing G/O emulsion.

### Reference Signs List

1a, 1b ··· holding part, 3 ··· sample addition port, 5 ··· support part, 7 ··· connection part, 9 ··· mouse skin, 10 ··· diffusion cell, 11 ··· phosphate-buffered saline, 20 ··· well.

## Claims

1. A gel-in-oil emulsion, comprising a gelled water-soluble polymer, an oil, and a surfactant.

2. The gel-in-oil emulsion according to claim 1, containing a water-soluble active ingredient in the gelled water-soluble polymer.

3. The gel-in-oil emulsion according to claim 2, wherein the water-soluble active ingredient is a growth factor.

4. The gel-in-oil emulsion according to any one of claims 1 to 3, wherein the gelled water-soluble polymer is a gelled polymer derived from a biological organism.

5. The gel-in-oil emulsion according to claim 4, wherein the polymer derived from a biological organism is a collagen or a gelatin.

6. A transdermal absorption preparation, comprising the gel-in-oil emulsion according to any one of claims 1 to 5.
